# EUROPÄISCHE PATENTANMELDUNG

(11) **EP 3 135 335 A1**
(43) Veröffentlichungstag der Anmeldung: **01.03.2017**
(21) Anmeldenummer: 16001559.0
(22) Anmeldetag: 14.07.2016
(51) Int. Cl.: A61N 1/36

(54) **MEDIZINISCHE VORRICHTUNG**

(30) Priorität: 29.08.2015 DE 102015011411
(71) Anmelder: cerboMed GmbH, 91052 Erlangen (DE)
(72) Erfinder: Hyca, Martin, 85221 Dachau (DE); Kreder, Dirk, 83607 Holzkirchen (DE); Hartlep, Andreas, 83607 Holzkirchen (DE)
(74) Vertreter: Gosdin, Michael

(57) **Zusammenfassung**

Die Erfindung betrifft eine medizinische Vorrichtung (1) zur Elektrostimulation eines Nervs des menschlichen Körpers, die eine Steuerungseinrichtung (5) umfasst, wobei die Steuerungseinrichtung (5) mit mindestens einem Display (6) in Verbindung steht, auf dem Informationen angezeigt werden können. Um eine besonders energieoptimierte Betriebsweise zu erlauben, sieht die Erfindung vor, dass das Display (6) zumindest abschnittsweise als reflektives Display ausgebildet ist, das von der Steuerungseinrichtung (5) ansteuerbar ist, wobei die Steuerungseinrichtung (5) ausgebildet ist, vorgegebene Daten bei einem Stromverlust zumindest vom Display (6) zu löschen, so dass diese Daten nicht im Display (6) sichtbar bleiben.

## Beschreibung

Die Erfindung betrifft eine medizinische Vorrichtung zur Elektrostimulation eines Nervs des menschlichen Körpers, die eine Steuerungseinrichtung umfasst, wobei die Steuerungseinrichtung mit mindestens einem Display in Verbindung steht, auf dem Informationen angezeigt werden können.

Bei medizinischen Vorrichtungen der gattungsgemäßen Art besteht die Notwendigkeit, dem Anwender der Vorrichtung gewisse Informationen bereitzustellen, wozu ein Display zum Einsatz kommt.

Eine Vorrichtung der eingangs genannten Art ist beispielsweise aus der DE 10 2010 054 165 B3 bekannt. Hier ist eine Vorrichtung zur transkutanen Stimulation des Vagusnervs des menschlichen Körpers beschrieben, deren Elektrodenkopf mit zwei Elektroden im Bereich der Cymba conchae angeordnet wird; eine solche Positionierung der Elektroden hat sich als vorteilhaft erwiesen. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Nachteilig ist, dass insbesondere bei einem schwachen Batteriestatus bzw. einem vollständigen Energieausfall auf dem Display keinerlei Hinweise mehr zu finden sind, beispielsweise darüber, was im weiteren vom Benutzer der Vorrichtung zu veranlassen ist.

Diverse weitere Lösungen, die der oben genannten Vorrichtung ähnlich sind, sind aus der DE 20 2012 103 226 U1, aus der DE 10 2013 021 175 A1, aus der DE 60 2004 004 675 T2, aus der DE 10 2013 106 864 A1, aus der DE 10 2010 052 710 A1 und aus der DE 10 2009 037 651 A1 bekannt.

Der vorliegenden Erfindung liegt daher die Aufgabe zugrunde, eine Vorrichtung der eingangs genannten Art bereitzustellen, mit der eine verbesserte und energieoptimierte Betriebsweise ermöglicht wird. Insbesondere soll es möglich werden, den Benutzer der Vorrichtung auch im Falle dessen mit gewissen Informationen zu versorgen, wenn es zu einem Ausfall der Stromversorgung in der Vorrichtung gekommen ist. Dabei sollen insbesondere Vorgaben des Datenschutzes in verbesserter Weise berücksichtigt werden können.

Die **Lösung** dieser Aufgabe durch die Erfindung ist dadurch gekennzeichnet, dass das Display zumindest abschnittsweise als reflektives Display ausgebildet ist, das von der Steuerungseinrichtung ansteuerbar ist, wobei die Steuerungseinrichtung ausgebildet ist, vorgegebene Daten bei einem Stromverlust zumindest vom Display zu löschen, so dass diese Daten nicht im Display sichtbar bleiben.

Besonders bevorzugt ist vorgesehen, dass die Vorrichtung eine solche zur Aufbringung eines transkutanen elektrischen Stimulationsreizes auf die Oberfläche eines Abschnittes des menschlichen Ohrs ist, die mindestens zwei Elektroden aufweist, wobei die Steuerungseinrichtung zur Einleitung eines über die Elektroden fließenden Stimulationsstroms ausgebildet ist.

Das Display ist bevorzugt ausgebildet, eine Anzeige auch ohne Anlegen einer Erhaltungsspannung dauerhaft anzuzeigen.

Die Steuerungseinrichtung ist gemäß einer bevorzugten Ausgestaltung der Erfindung ausgebildet, die Beaufschlagung des Displays mit einer Spannung abzuschalten, wenn nach einer vorgegebenen Zeit keine Änderungen der Ansteuerung des Displays erfolgt. Auf diese Art und Weise kann vorteilhaft Energie eingespart und die Batterie geschont werden. Dennoch bleibt die gewünschte Anzeige auf dem Display erhalten.

Gemäß einer weiteren Ausgestaltung der Erfindung ist vorgesehen, dass neben dem erläuterten Display ein weiteres Display oder ein Displayabschnitt angeordnet ist, das oder der eine Anzeige nur bei Aufbringung einer Spannung durch die Steuerungseinrichtung bewerkstelligt. Das weitere Display oder der weitere Displayabschnitt kann dabei aus einem LED-Bereich besteht oder einen solchen aufweisen.

Es kann dabei insbesondere auch vorgesehen sein, dass sensible persönliche Daten des Benutzers der Vorrichtung auf dem bzw. vom Display nach Abschalten des Stroms bzw. bei Stromausfall nicht endgültig gelöscht, sondern lediglich unterdrückt werden und nach Wiedereinschalten des Stroms erneut verfügbar sind. Indes erfolgt erfindungsgemäß eine Unterdrückung der Sichtbarkeit der sensiblen persönlichen Daten auf dem Display bei Stromverlust, während andere Daten dort stehen bleiben können. Beim Wiedereinschalten des Stroms ist die erneute Sichtbarkeit aller Daten gegeben.

Zu dem zum Einsatz kommenden reflektiven Display ist folgendes anzumerken:
Ein solches wird gelegentlich auch als "elektronisches Papier" bezeichnet; gelegentlich wird hier auch der Begriff "E-Ink" verwendet. Es geht dabei um eine Anzeigeart, mit der eine Nachbildung von Tinte bzw. Farbe auf einem Papier bewerkstelligt wird. Das reflektive Display reflektiert Licht wie normales Papier, so dass es sich um eine passive, d. h. nicht-leuchtende Anzeige handelt.

Die Information, d. h. Texte und/oder Grafiken, werden dauerhaft auf dem reflektiven Display angezeigt, ohne dass hierfür eine Erhaltungsspannung an das Display angelegt werden muss. Allerdings ist es - wie bei einem klassischen Display, zum Beispiel bei einem LCD-Display - natürlich möglich, die Darstellung bzw. Anzeige zu ändern, wozu eine entsprechende Spannung von der Steuerungseinrichtung an das Display angelegt wird.

Die Wirkungsweise des in Rede stehenden reflektiven Displays kann auf der sogenannten Elektrophorese basieren. Bei diesem Verfahren werden beispielsweise Mikrokapseln eingesetzt, in denen elektrisch geladene weiße Teilchen in gefärbtem Öl schwimmen. Durch die Polarität eines angelegten elektrischen Feldes wandern die weißen Partikel an die Oberseite der Mikrokapsel und sind somit für den Betrachter sichtbar; oder aber sie wandern an die Unterseite, so dass der Betrachter an diesem Punkt die dunklere Farbe des Öls sieht.

Ein reflektives Display, das auf dieser Basis arbeitet, enthält Mikrokapseln mit einem mittleren Durchmesser von beispielsweise 40 µm, die positiv geladene weiße Partikel und negativ geladene schwarze Partikel in einem transparenten zähflüssigen Polymer enthalten. Die Darstellung wird durch kurzzeitiges Anlegen einer elektrischen Spannung verändert und bleibt dann mehrere Wochen lang oder noch länger stabil. Die Verwendung derartiger Mikrokapseln erlaubt auch den Einsatz eines flexiblen Kunststoffs anstelle von Glas als Trägermaterial.

Die Ansteuerung der Bildpunkte geschieht bei Segmentanzeigen mittels passiver transparenter Elektroden und bei Matrixdisplays über eine TFT-Aktiv-Matrix, wie sie auch bei LC-Bildschirmen verwendet wird.

Gemäß der vorliegenden Erfindung wird somit nunmehr das Display der Stimulationsvorrichtung mit einem "e-Paper" bestückt, was als solches beispielsweise bei e-Books bekannt und eingesetzt wird. Problemlos ist hierbei der Einsatz von schwarzem oder rotem Farbstoff als Anzeigematerial, das durch Spannungsänderungen in einem Zwischenraum hin und her geschoben werden kann, um die entsprechenden Bereiche des Displays sichtbar bzw. unsichtbar zu machen. Sehr vorteilhaft ist hierbei, dass die zuletzt bestehende Anzeige auch bei Abschalten des Stroms über längere Zeit (wie erwähnt im Bereich von Wochen oder gar Monaten) bestehen bleibt. Dies hat insbesondere bei der Anwendung für einen Stimulator für die transkutane Stimulation des Vagusnervs besondere Vorteile.

Zunächst bleibt bei einer nicht beabsichtigten Stromunterbrechung der zuletzt angezeigte Wert im Display sichtbar, beispielsweise auch eine Meldung, die den Nutzer anweist, welche Schritte er nun unternehmen muss, um im weiteren die benötigte Stimulation zu bewerkstelligen; oder es werden zuletzt geltende Stimulationsparameter angezeigt. Bei einem bislang eingesetzten Display bei einer Stimulationsvorrichtung ist dies nicht möglich, da die Anzeige bei unterbrochener Stromzufuhr im selben Moment komplett erlischt.

Gemäß der Erfindung ist durch entsprechende Programmierung vorgesehen, dass sensible persönliche Daten bei einem Stromverlust automatisch gelöscht werden und dann eben doch nicht im Display sichtbar bleiben.

Bei der Programmierung eines entsprechenden Algorithmus kann weiterhin vorgesehen werden, dass die Stromzufuhr zum Display unterbrochen wird, wenn über einen gewissen Zeitraum (d. h. über eine definiert vorgegebene Zeit) keine Änderungen der Bestromung des Displays erfolgt. Auf diese Art und Weise kann Strom gespart werden, der Akku der Stimulationsvorrichtung hält länger. Dies ist besonders wichtig zur Aufrechterhaltung einer gleichbleibend guten Stimulationsqualität. Ein solcher Algorithmus kann auch dafür sorgen, dass das Display dann wieder Strom erhält, wenn sich ein Wert oder eine Darstellung ändert. Falls nicht, bleibt die letzte Anzeige quasi "eingefroren".

Vorteilhaft ist bei der vorgeschlagenen Ausgestaltung des Displays, dass auch bei Sonneneinstrahlung ein solches Display gut lesbar ist und einen guten Kontrast bietet. Die Ablesbarkeit des Displays ist somit gewährleistet.

In der Nacht kann das Display, wie in jeder Digitaluhr, von der Seite her einfach und bequem extern beleuchtet werden. Dies ist auch erforderlich, weil das Display nicht von sich aus leuchtet und auch nicht hinterleuchtet werden kann.

In vorteilhafter Weise kann auch vorgesehen werden, dass eine Kombination des vorgeschlagenen e-Paper-Displays mit LED-Anzeigen und konventionellen Farbdisplays erfolgt, um jeweils deren Vorteile zusammen nutzen zu können. Das Display wäre in diesem Fall bevorzugt in zwei oder drei Felder aufgeteilt, die mit unterschiedlicher Technologie arbeiten bzw. angesteuert werden.

Generell kann die vorgeschlagene Lösung bei allen elektromedizinischen Geräten eingesetzt werden. Besonders bevorzugt kann sie bei elektrischen Stimulationsgeräten, wie beispielsweise Reizstromgeräten, aber auch bei implantierten Stimulationsgeräten (mit externer Basisstation) oder bei transkutanen Neurostimulationsgeräten verwendet werden. Besonders bevorzugt ist der Anwendungsfall von TENS- und von t-TNS-Geräten vorgesehen und insbesondere die Anwendung bei transkutanen Stimulationsgeräten zur Stimulation des Vagusnervs im Bereich des Ohrs.

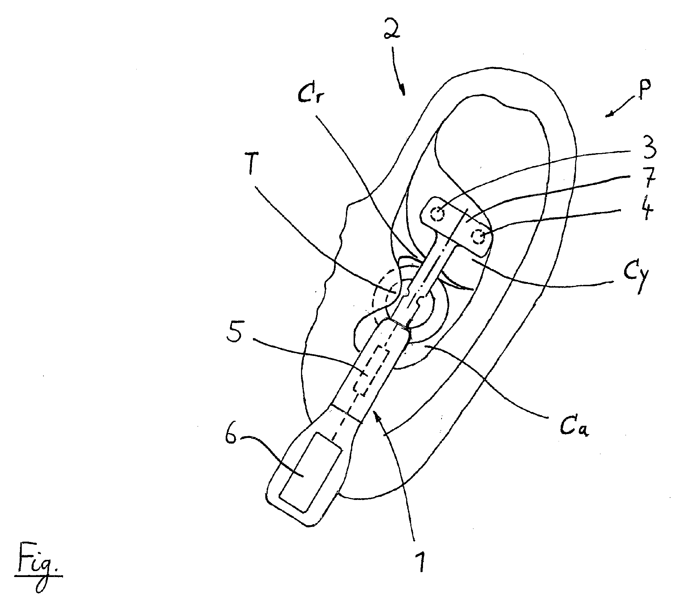

In der Zeichnung ist ein Ausführungsbeispiel der Erfindung dargestellt. Die einzige Figur zeigt schematisch die Ansicht einer Pinna (Ohrmuschel), in die eine Vorrichtung zur Aufbringung eines transkutanen Stimulationsreizes eingesetzt ist.

In der Figur ist eine medizinische Vorrichtung 1 in Form einer Vorrichtung zur transkutanen Stimulation eines Abschnitts des menschlichen Ohrs 2 skizziert. Die Vorrichtung 1 weist eine Haltestange auf, die in einem Basisteil längsverschieblich ist, das auch eine Steuerungseinrichtung 5 umfasst. Am Basisteil ist ein Auflageteil angeordnet. Die generelle Positionierung der Vorrichtung 1 im Ohr 2 ergibt sich aus der Markierung der wesentlichen Bereiche des Ohrs 2, nämlich der Pinna P mit dem Cavum conchae Ca, der Cymba conchae Cy, dem Tragus T und dem Crus helicis Cr.

Am einen Ende der Haltestange ist ein Elektrodenträger 7 angeordnet, der zwei Elektroden 3 und 4 aufweist, zwischen denen zwecks transkutaner Stimulation eine Potentialdifferenz aufgebaut wird. Hiermit wird ein Stimulationsreiz erzeugt.

Insoweit entspricht die Vorrichtung 1 zunächst vorbekannten Lösungen, wobei insbesondere und ausdrücklich auf die DE 10 2010 054 165 B3 der Patentinhaberin Bezug genommen wird, wo eine solche Vorrichtung näher erläutert ist.

Die Vorrichtung 1 ist demnach ausgebildet, um im Bereich des Vagusnervs am Ohr 2 der die Vorrichtung benutzenden Person angebracht zu werden. Damit kann eine transkutane Stimulation des Vagusnervs vorgenommen werden.

Wesentliches Element der vorliegenden Vorrichtung ist ein Display 6, das als reflektives Display ausgebildet ist. Demgemäß ist es möglich - und es wird insofern auf die obigen Erläuterungen Bezug genommen -, dass eine Darstellung auf dem Display 6 erfolgt, ohne dass dafür eine ständige Bestromung des Displays 6 durch die Steuerungseinrichtung 5 erforderlich ist.

Damit lassen sich die oben genannten Vorteile bei der Stimulation erreichen und insbesondere eine besonders energieoptimierte Betriebsweise realisieren.

Im dargestellten Ausführungsbeispiel ist das Display 6 direkt an der Vorrichtung 1 angeordnet. Es ist natürlich aber auch möglich, dass die Stimulationsvorrichtung 1 zweiteilig ausgebildet ist und der Bereich, der das Display 6 aufweist, trennbar von demjenigen Teil ist, der die Elektroden 3, 4 aufweist. In diesem Falle kann eine Kommunikation zwischen den Teilen per Kabel oder drahtlos erfolgen.

### Bezugszeichenliste:

- 1: medizinische Vorrichtung (Vorrichtung zur transkutanen Stimulation)
- 2: Ohr
- 3: Elektrode
- 4: Elektrode
- 5: Steuerungseinrichtung
- 6: Display
- 7: Elektrodenträger

- Ca: Cavum conchae
- Cy: Cymba conchae
- T: Tragus
- Cr: Crus helicis
- P: Pinna

## Patentansprüche

1. Medizinische Vorrichtung (1) zur Elektrostimulation eines Nervs des menschlichen Körpers, die eine Steuerungseinrichtung (5) umfasst, wobei die Steuerungseinrichtung (5) mit mindestens einem Display (6) in Verbindung steht, auf dem Informationen angezeigt werden können,
**dadurch gekennzeichnet,**
**dass** das Display (6) zumindest abschnittsweise als reflektives Display ausgebildet ist, das von der Steuerungseinrichtung (5) ansteuerbar ist, wobei die Steuerungseinrichtung (5) ausgebildet ist, vorgegebene Daten bei einem Stromverlust zumindest vom Display (6) zu löschen, so dass diese Daten nicht im Display (6) sichtbar bleiben.

2. Medizinische Vorrichtung nach Anspruch 1, **dadurch gekennzeichnet, dass** es eine Vorrichtung (1) zur Aufbringung eines transkutanen elektrischen Stimulationsreizes auf die Oberfläche eines Abschnittes des menschlichen Ohrs (2) ist, die mindestens zwei Elektroden (3, 4) aufweist, wobei die Steuerungseinrichtung (5) zur Einleitung eines über die Elektroden (3, 4) fließenden Stimulationsstroms ausgebildet ist.

3. Medizinische Vorrichtung nach Anspruch 1 oder 2, **dadurch gekennzeichnet, dass** das Display (6) ausgebildet ist, eine Anzeige ohne Anlegen einer Erhaltungsspannung dauerhaft anzuzeigen.

4. Medizinische Vorrichtung nach einem der Ansprüche 1 bis 3, **dadurch gekennzeichnet, dass** die Steuerungseinrichtung (5) ausgebildet ist, die Beaufschlagung des Displays (6) mit einer Spannung abzuschalten, wenn nach einer vorgegebenen Zeit keine Änderungen der Ansteuerung des Displays (6) erfolgt.

5. Medizinische Vorrichtung nach einem der Ansprüche 1 bis 4, **dadurch gekennzeichnet, dass** neben dem Display (6) ein weiteres Display oder ein Displayabschnitt angeordnet ist, das oder der eine Anzeige nur bei Aufbringung einer Spannung durch die Steuerungseinrichtung (5) bewerkstelligt.

6. Medizinische Vorrichtung nach Anspruch 5, **dadurch gekennzeichnet, dass** das weitere Display oder der weitere Displayabschnitt aus einem LED-Bereich besteht oder einen solchen aufweist.
